# EUROPEAN PATENT APPLICATION

(11) **EP 0 775 741 A1**
(43) Date of publication of application: **28.05.1997**
(21) Application number: 95203207.6
(22) Date of filing: 22.11.1995
(51) Int. Cl.: C11D 17/04, C11D 3/50, C11D 3/43, C11D 17/00, A61L 9/01

(54) **Lavatory freshener/cleaner system**

(71) Applicant: Sara Lee/DE N.V., 3532 AA Utrecht (NL)
(72) Inventor: Van Walsum, Arnoud,, 3335 DE Zwijndrecht (NL)
(74) Representative: Smulders, Theodorus A.H.J., Ir.

(57) **Abstract**

The invention is directed to a lavatory freshener/cleaner system, comprising a container for a liquid lavatory freshener/cleaner composition, dispenser means of the blotter type connected to the said container, said lavatory freshener/cleaner composition being contained in said container, having a viscosity at room temperature of 10 to 2000 mPa.s, and comprising:
a. 1 to 25 wt.% of perfume,
b. 10 to 50 wt.% of anionic or non-ionic surfactant,
c. 1 to 20 wt.% of non-evaporating, water soluble evaporation regulator, and
d. balance solvent.

## Description

The present invention is directed to a lavatory freshener/cleaner system, more in particular to a lavatory freshener/cleaner system wherein a liquid composition is held in a container which is provided with dispenser means of the blotter type to provide automatically an amount of freshener/cleaner composition to the lavatory, each time the lavatory is flushed.

In freshening and cleaning of lavatories various systems are known. One type of system is based on a solid block of a freshening/cleaning composition, which is put into the toilet bowl underneath the rim thereof. Each time the lavatory is flushed, the water dissolves part of the block, resulting in a freshening and cleaning function.

Other systems are known, which are based on freshening/cleaning compositions present in the water reservoir or which are based on electronic signals dispensing an amount of composition once activated.

The present invention deals with a lavatory freshener/cleaner system based on a container for a liquid freshener/cleaner composition, which container is in contact with dispenser means of the blotter type. This system is designed so that it can be located in the lavatory bowl, preferably underneath the rim thereof, so that each time the lavatory is flushed water comes into contact with the blotter. Accordingly, each flushing results therein that a certain, small amount of lavatory freshener/cleaner composition is introduced into the lavatory bowl, resulting in a freshening and cleaning action. Additionally, this system provides a continuous generation of perfume, even when the lavatory is not in use for some time.

A lavatory freshener/cleaner composition destined for use in this system must meet some very strict requirements. In the first place it is essential that the system is liquid and remains liquid during the entire life thereof, even under the various temperature conditions maintained in lavatories. Especially after some time the composition may change due to selective evaporation of certain components, which may not result in the generation of solid particles or in the phase separation in the composition. Additionally, it is essential that the composition contains a relatively high amount of perfume, as otherwise the freshening action will be insufficient due to the extremely small volumes generated through the blotter at each flushing.

Other requirements on the system are that the evaporation from the blotter when there is no flushing may not result in a precipitation of components on the blotter, resulting in partial or total blocking of the supplied capacity of the blotter. On the other hand, the rheological behaviour of the freshener/cleaner system must be such that the system is only removed from the blotter by evaporation or flushing. It is not allowed that the composition drips from the blotter into the bowl. This aspect is especially of importance when the container with the composition is located above the blotter, as will generally be the case.

Accordingly, it is an object of the present invention to provide a system for lavatory freshening and cleaning, as well as a composition thereof, which is able to meet all those, partly conflicting requirements.

According to the invention there is provided a lavatory freshener/cleaner system, comprising a container for a liquid lavatory freshener/cleaner composition, dispenser means of the blotter type connected to the said container, said lavatory freshener/cleaner composition being contained in said container, having a viscosity at room temperature of 10 to 2000 mPa.s, and comprising:
a. 1 to 25 wt.% of perfume,
b. 10 to 50 wt.% of anionic or non-ionic surfactant,
c. 1 to 20 wt.% of non-evaporating, water soluble evaporation regulator, and
d. balance solvent.

Surprisingly it has been found that the composition as defined herein is able to meet all these criteria, resulting in a superior freshener and cleaning system.

According to a preferred embodiment the system comprises:
a. 5 to 15 wt.% of perfume,
b. 15 to 40 wt.% of anionic or non-ionic surfactant,
c. 5 to 20 wt.% of evaporation regulator, and
d. balance solvent.

Important aspects of the present invention are accordingly the amount of perfume, which is required to generate sufficient freshening function. In this respect it is to be noted that in some instances chlorine bleaches may also be considered as perfume. Further, it is to be noted that the amount of perfume is calculated on the pure perfume, without solvents, diluents or surfactants. As the perfumes are generally oil-soluble products, it is very difficult to incorporate them in a one-phase freshener/cleaner system, without problems occurring due to phase separation and/or insufficient distribution. However, by the provision of a high amount of anionic or non-ionic surfactant in combination with the non-evaporating, water soluble evaporation regulator, it has become possible to micro-emulsify/dissolve/disperse the perfume in such a way that there are no, or only small, problems with respect to the distribution of perfume. Additionally, the perfume is dispersed continuously and almost at the same level throughout the life of the lavatory freshener/cleaner system.

The surfactant used in the invention does not only provide the good distribution of the perfume, but is also of importance in the cleaning function of the system. These combined functions and the requirement that the surfactant is present in relatively high amounts, up to 50 wt.% makes the choice of the specific surfactant somewhat critical. Preferred surfactants are non-ionics, especially those based on alkoxylated fatty acids and alkoxylated fatty acid derivatives and alkoxylated fatty alcohols, such as fatty acid ether sulfates and ethoxylated fatty acid oxo-alcohols. Most preferred is an ethoxylated, sulfated C₁₂₋₁₄ ether fat-alcohol, which can be used in an amount of 10 to 50 wt.%, based on the total weight of the composition. It is to be noted that these types of preferred surfactants also do not give problems with respect to gelation of the system, more in particular when used in combination with the evaporation regulator.

The evaporation regulator has also a multiple function in the system, as it provides on the one hand a controlled release of the perfume at the blotter surface, when the lavatory is not in use. On the other hand, it prevents the precipitation of solid components and/or the phase separation into two liquid phases of the composition, both initially and during the use of the composition, when certain components may have been evaporated at relatively higher rate, thus changing the composition of the system. These evaporation regulators are water-soluble, whereas they substantially do not evaporate at room temperature and atmospheric pressures, that is under the usual conditions that the freshener/cleaner systems are used. Examples of these evaporation regulators are the various glycols, glycolethers, alcohols, sugars and polyethers, more preferably glycerine, mono-, di-, or tri-propylene glycol, sorbitol, polypropylene glycol and/or polyethylene glycol. Most preferred is the use of mono-propyleneglycol. Generally one can state that humectants as used in some of the body care systems may be used as evaporation regulator in these systems.

A further important requirement on the composition is the rheological behaviour. In the first place it is important that it has a viscosity, at room temperature, of 10 to 2000 mPa.s, determined by the use of a Brookfield viscosimeter. Additionally, it is preferred that the composition has a yield point or yield value, which means that to at least some degree it shows a thixotropic behaviour. The actual yield value can be determined on the basis of the final system, more in particular taking into account the type of blotter used and the pore size thereof.

In order to provide the required rheological behaviour various options are open to the skilled person. In the first place it is possible, by careful selection of combinations of surfactants to meet especially the viscosity requirements. As an alternative it is also possible to add a thickener or rheological behaviour modifier to the system. Examples thereof are the various cellulose derivatives, gums and synthetic polymers, but also silica. Examples of these types of compounds are hydroxyethylpropyl cellulose, hydroxypropyl cellulose, hydroxybutyl methyl cellulose, hydroxypropyl methyl cellulose, hydroxyethyl cellulose and sodium carboxymethyl cellulose as the cellulose derivatives. Examples of gums are xanthan gum, Irish moss, caragheenen, alginates, gelatin, agar-agar, and gum tragacanth. Synthetic polymers such as (meth)acrylates and the various vinylpyrrolidone homo- and copolymers can also be used.

The matrix material of the system is the solvent, which is present as the balance of the components up to 100 %. Generally, it will be preferred that the amount of solvent is between 20 and 60 wt.% of the composition. As solvent the most preferred is water, although other solvent systems, such as mixtures of water and alcohols, or mixtures of alcohols, can also be used, provided that the system is homogeneous.

Apart from the above essential components the composition may contain other additives which are destined to improve the function of the system. Examples thereof are neutralising agents, sequestering agents, bleaching agents, disinfecting agents, biocides, preservatives, soil repellants, soil release agents, odour neutralisers, insecticides or insect-repellants, enzymes, coloring agents and the like.

Neutralising agents may be present when it is required that the pH of the composition is adjusted to a certain level. Generally, the pH of the system may range from acid through neutral to basic. The actual choice will depend on the composition and the properties required of the system. Suitable neutralising agents are triethanol amine, citric acid, oxalic acid, and the like.

Sequestering agents may be added to the system to provide activity against the precipitation of calcium salts, such as calcium carbonate, or for the removal of precipitated calcium salts. Examples are the various commercially available fosfonates.

Bleaching agents may be used in the system as active component, optionally in combination with perfume, although, as indicated above, under some circumstances bleaching agents may be used alone, instead of perfume. This is, however, not preferred.

Disinfecting agents for the lavatory bowl may also be added. Examples thereof are hypochlorite, peroxide, isocyanurates, quaternary ammonium compounds, certain aldehydes and their donors. Generally, one also uses preservatives in the composition, to prevent deterioration thereof through microbiological attack. Examples are various ketones like those cited in the positive EU list for preservation agents. Finally also enzymes, coloring agents and other functional components may be used in the system. The amounts of these agents will depend on the actual application and can easily be determined by the skilled person. Generally, the total amount of these additional agents will not exceed 10 wt.% of the total weight of the composition.

Finally, the present invention is also directed to a method of freshening a lavatory when flushing by use of the system as disclosed hereinabove. Additionally, the invention also concerns the actual freshener/cleaner composition having a viscosity at room temperature of 10 to 2000 mPa.s, and comprising:
a. 1 to 25 wt.% of perfume,
b. 10 to 50 wt.% of anionic and/or non-ionic surfactant,
c. 1 to 20 wt.% of non-evaporating, water soluble evaporation regulator, and
d. balance solvent.

The invention is now elucidated on the basis of the following Examples.

### Example 1

| Anionic system | |
|---|---|
| Component | wt.% |
| Fragrance | 10 |
| Mono propylene glycol | 5 |
| Marlinat® 242/90/T | 25 |
| Dequest® 2010 | 0.5 |
| TEA | 0.5 |
| Kelzan® T | 0.2 |
| Preservative | 0.2 |
| Water | 58.6 |

The composition is prepared by cold mixing fragrance, glycol and Marlinat until a clear solution has been obtained. The remaining components are also cold mixed until a clear solution has been obtained. Both solutions are subsequently mixed with each other and filled into a blotter type lavatory freshener/cleaner system.

### Example 2

| Nonionic system | |
|---|---|
| Component | wt.% |
| Fragrance | 10 |
| Mono propylene glycol | 5 |
| Lutensol® AO 10 | 12 |
| Lutensol® ON 80 | 6 |
| Tween® 80 | 6 |
| Dequest® 2010 | 0.5 |
| TEA | 0.5 |
| Kelzan® T | 0.2 |
| Preservative | 0.2 |
| Water | 59.6 |

The composition is prepared by cold mixing fragrance and Lutensols until a clear solution has been obtained. The remaining components are also cold mixed until a clear solution has been obtained. Both solutions are subsequently mixed with each other and filled into a blotter type lavatory freshener/cleaner system.

## Claims

1. Lavatory freshener/cleaner system, comprising a container for a liquid lavatory freshener/cleaner composition, dispenser means of the blotter type connected to the said container, said lavatory freshener/cleaner composition being contained in said container, having a viscosity at room temperature of 10 to 2000 mPa.s, and comprising:
a. 1 to 25 wt.% of perfume,
b. 10 to 50 wt.% of anionic or non-ionic surfactant,
c. 1 to 20 wt.% of non-evaporating, water soluble evaporation regulator, and
d. balance solvent.

2. System according to claim 1, wherein the composition further comprises:
e. 0,1 to 15 wt.% of at least one thickener.

3. System according to claim 1 or 2, wherein the composition comprises:
a. 5 to 15 wt.% of perfume,
b. 15 to 40 wt.% of anionic or non-ionic surfactant,
c. 5 to 20 wt.% of evaporation regulator, and
d. balance solvent.

4. System according to claim 2 or 3, wherein the thickener is selected from the group of cellulose derivatives such as hydroxyethylpropyl cellulose, hydroxybutyl methyl cellulose, hydroxypropyl cellulose, hydroxypropyl methyl cellulose, hydroxyethyl cellulose and sodium carboxymethyl cellulose, gums, such as xanthan gum, caragheenen, alginates, gelatin, agar-agar, Irish moss and gum tragacanth, starch and synthetic polymers such as (meth)acrylates, polyvinylpyrrolidone and vinylpyrrolidone copolymers.

5. System according to claim 1-4, wherein the surfactant is selected from the group consisting of alkoxylated fatty acids alkoxylated fatty acid derivatives and alkoxylated fatty alcohols.

6. System according to claim 5, wherein the surfactant is selected from the group of fatty acid ether sulfates and ethoxylated fatty alcohols, more in particular ethoxylated fatty acid oxo-alcohols, such as ethoxylated, sulfated C₁₂₋₁₄ ether fat-alcohol.

7. System according to claim 1-6, wherein the evaporation regulator has been selected from the group of glycols, glycolethers, alcohols, sugars, and polyethers.

8. System according to claim 7, wherein the evaporation regulator has been selected from the group of glycerine, mono-, di-, or tri-propylene glycol, sorbitol, polypropylene glycol and/or polyethylene glycol.

9. System according to claim 1-8, wherein the solvent is selected from water and mixtures of water and at least on alcohol.

10. System according to claim 1-9, wherein the composition additionally contains at least one active component.

11. System according to claim 10, wherein the said component has been selected from the group of neutralising agents, sequestering agents, bleaching agents, disinfecting agents, biocides, preservatives, soil repellants, soil release agents, odour neutralisers, insecticides or insect-repellants, enzymes, coloring agents and the like.

12. System according to claim 1-11, wherein the blotter type dispenser is destined to come into contact with flushing water when the lavatory is flushed.

13. Method of freshening a lavatory when flushing, said method providing a system according to claim 1-12 in the lavatory, which system comes into contact with flushing water, when the lavatory is flushed.

14. Lavatory freshener/cleaner composition having a viscosity at room temperature of 10 to 2000 mPa.s, and comprising:
a. 1 to 25 wt.% of perfume,
b. 10 to 50 wt.% of anionic or non-ionic surfactant,
c. 1 to 20 wt.% of non-evaporating, water soluble evaporation regulator, and
d. balance solvent.
